# EUROPEAN PATENT SPECIFICATION

(11) **EP 1 987 802 B1**
(45) Date of publication and mention of the grant of the patent: **23.06.2010**
(21) Application number: 08155074.1
(22) Date of filing: 24.04.2008
(51) Int. Cl.: A61F 2/01

(54) **Removable Medical Filter with Partial Baskets**
Abnehmbarer medizinischer Filter mit Teilkörben
Filtre médical extractible doté de paniers partiels

(30) Priority: 01.05.2007 US 742712
(43) Date of publication of application: 05.11.2008
(73) Proprietor: Cordis Corporation, Miami Lakes, FL 33014 (US)
(72) Inventor: Fleming, James A. III, Bethlehem, Pennsylvania 18020 (US)
(74) Representative: Belcher, Simon James

(56) References cited:
- WO-A-2004/058110
- DE-A1- 3 429 850
- US-A1- 2002 026 211

## Description

The present invention relates to medical filters which are intended to be placed inside a blood vessel or other body passage for the purpose of intercepting thrombus or particles.

Medical filters, including vena cava filters, are emplaced inside blood vessels or other body passages to trap unwanted particles. When used in a blood vessel, a medical filter should be effective to entrap thrombus, clots or other dangerous coagulations or particulate matter in the blood while allowing free flow of blood through the filter in the vessel. While in some cases it is desirable to leave a medical filter in place indefinitely, in other cases it is desirable to retrieve the filter after it has been in place for a period of time. Such retrieval can be difficult. For example, when a vascular filter is placed within a vessel of the human body for any significant length of time, the inner lining of the vessel will grow around the portion of the device that is in contact with the tissue and encapsulate it. Tissue does not attach itself directly to materials such as Nitinol or stainless steel and it is known to make medical filters out of materials. However, depending upon the design of the filter, removal can still be difficult if there is mechanical interference between the tissue and elements of the filter. Corners, loops or other features of the filter must not interfere with tissue when the filter is retrieved in order for the retrieval to be atraumatic.

Another important feature desirable in a medical filter is that it be able to capture unwanted particles such as embolized blood clots without becoming occluded. It would also be desirable in some cases to have a vena cava or other medical filter designed to filter over a longer length than in a conventional filter to increase its clot capturing effectiveness while decreasing the risk of caval occlusion.

A medical filter is described in DE 3429850.

It is important that medical filters be resistant to migration. This is particularly the case with vascular filters. Thus, it is highly desirable that the filter resist movement in a vessel after it has been deployed yet be readily retrievable without tearing the tissue of the vessel. Of course, there are several other characteristics which are generally desirable in medical filters. For example, the filter should be of a design which can be deployed in a proper position in the blood vessel or body passage with minimal trauma to the patient and of a design which does not become entangled during its deployment. It is also desirable that the filter be adapted to center in the vessel and to properly adjust to the size of the vessel.

Although vascular and other medical filters are known in the art and have been found to be effective, the requirements of such filters can be high and there remains room for improved designs. In particular, there remains room for improved filters which exhibit the above-mentioned characteristics and yet can be economically manufactured. Accordingly, the present invention provides a medical filter which is suitable for placement in a blood vessel or body passage for an extended duration without migrating in the vessel or body passage and yet can be removed or retrieved with minimal trauma to the tissue of the blood vessel or body passage.

Furthermore, the filter of this invention is effective for long duration and is resistant to occlusion.

In accordance with the present invention, a preferred embodiment of the present invention is an elongated medical filter which has a longitudinal axis, is radially expandable and compressible and is intended for placement inside a tubular body passage, such as a blood vessel, of a patient. In expanded form, the medical filter comprises:
(A) a longitudinally extending spine with a retrieval element at one end thereof;
(B) a plurality of truncated filter baskets spaced longitudinally on said spine, each of truncated baskets defined by a plurality of individual arms extending radially outwardly from said spine, each of said truncated baskets being rotated about said longitudinal axis with respect to adjacent truncated filter baskets.

In one preferred embodiment of the present invention, the arms of each truncated filter basket are radially spaced about 180 degrees about said longitudinal axis. In another preferred embodiment of the present invention each of said arms has a free end extending generally axially and radially inwardly. In another preferred embodiment of the present invention, each of said arms extends radially inwardly at an angle approximately transverse to said longitudinal axis. In yet another preferred embodiment of the present invention the spine is comprised of a flexible material.

Further understanding of the present invention will be had from the following description taken in conjunction with the accompanying drawings and appended claims.

Embodiments of the invention will now be described by way of example, with reference to the accompanying drawings, in which:
FIG.1 is a side elevational view of a preferred embodiment of a medical filter of the present invention shown in expanded form;
FIG. 2 is a perspective view, broken away, showing a truncated basket of the preferred embodiment of Figure 1;
FIG. 3 is a perspective view, broken away, showing an alternative preferred embodiment of a medical filter of the present invention;
FIG. 4 is a somewhat schematic view illustrating the use of the preferred embodiment of Figure 1 in a blood vessel;
FIG.5 is a somewhat schematic view illustrating the use of a short version of the preferred embodiment of Figure 1 above or below the renal veins of the inferior Vena Cava;
FIG. 6 is somewhat schematic view illustrating the use of a long version of the preferred embodiment of Figure 1 across the renal veins of the Inferior Vena Cava; and
FIG. 7 is a somewhat schematic view illustrating the use of an alternative preferred embodiment of Figure 1 in the Inferior Vena Cava to avoid interference with the renal arteries.

The following description of the preferred embodiments of the present invention is intended to be merely illustrative in nature, and as such, is not intended to limit in any way the present invention, its application, or uses. Numerous modifications may be made by those skilled in the art without departing from the scope of the invention. For example, the preferred embodiments of the present invention are described in conjunction with a blood vessel but the preferred embodiments may also be used in other body passages.

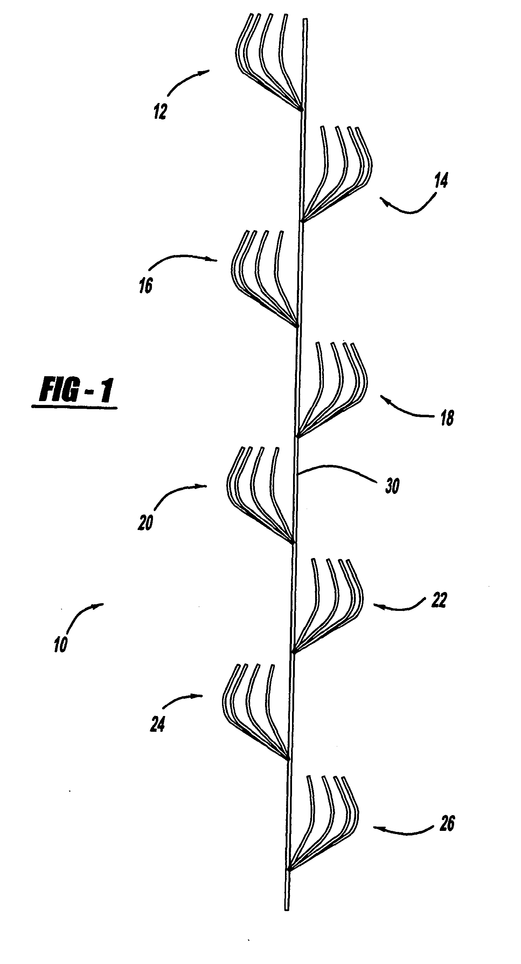

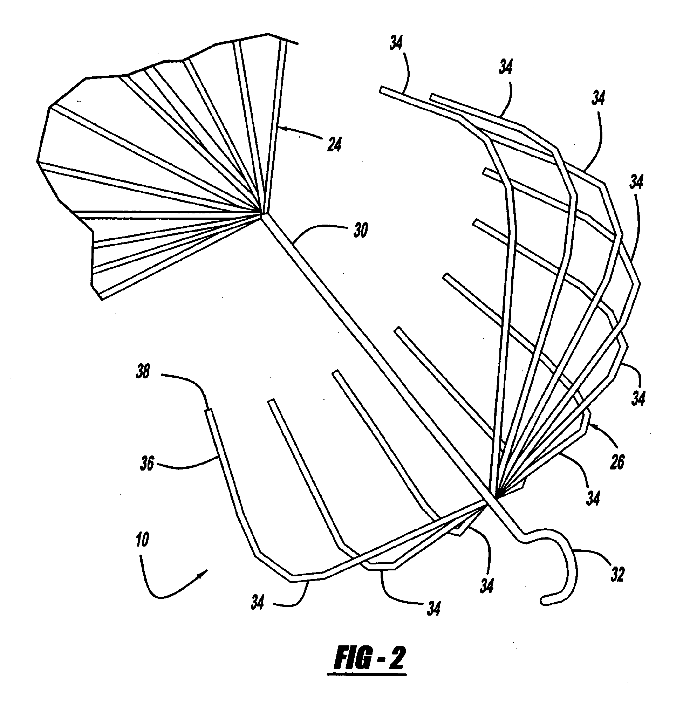

Now referring to Figures 1 and 2, a preferred embodiment of a medical filter of the present invention is shown and indicated generally by the numeral 10. Filter 10 is made of a resilient material, preferably a shape memory material, which tends to expand to the form illustrated in Figures 1 and 2 but can be compressed radially to a smaller diameter to be carried in the lumen of a suitable delivery catheter (not shown) as is conventional in the art.

Generally speaking medical filter 10 is radially expandable and compressible and is intended for placement inside a tubular body passage, such as a blood vessel, of a patient. It is contemplated that medical filter 10 will be compressed and placed into the lumen of a delivery catheter and then delivered to the desired site in a blood vessel or other body cavity whereupon filter 10 will be ejected from the catheter and allowed to self expand in position if the vessel or cavity. Figures 1 and 2 illustrate filter 10 in expanded form. In expanded form, medical filter 10 broadly comprises a plurality of truncated baskets 12, 14, 16, 18, 20, 22, 24, and 26 which are attached along spine 30 which has retrieval element 32 at one longitudinal end thereof.

Truncated basket 26 is defined by a plurality of arms 34 extending from spine 12. As is best shown in Figure 2, each arm 34 extends generally radially outwardly from spine 30 to provide a segment generally transverse to flow through filter 10 and each arm 34 has a free end segment 36 which extends generally axially with free end 38 positioned slightly radially inwardly so as not to interfere with an associated wall of a vessel or body passage. As viewed from an axial direction, arms 34 are spaced about 180° radially about spine 12 to define a truncated or half basket shape.

Truncated baskets 12, 14, 16, 18, 20, 22, 24, and 26 are of a configuration identical to that of basket 12 but are positioned radially about Spine 12 to be out of alignment with adjacent baskets. As will be discussed in more detail below, the truncated design and radial alignment, or more specifically, the radial non-alignment of truncated baskets allows for flow of fluid through filter 10 even if the baskets are completely blocked with filtered material.

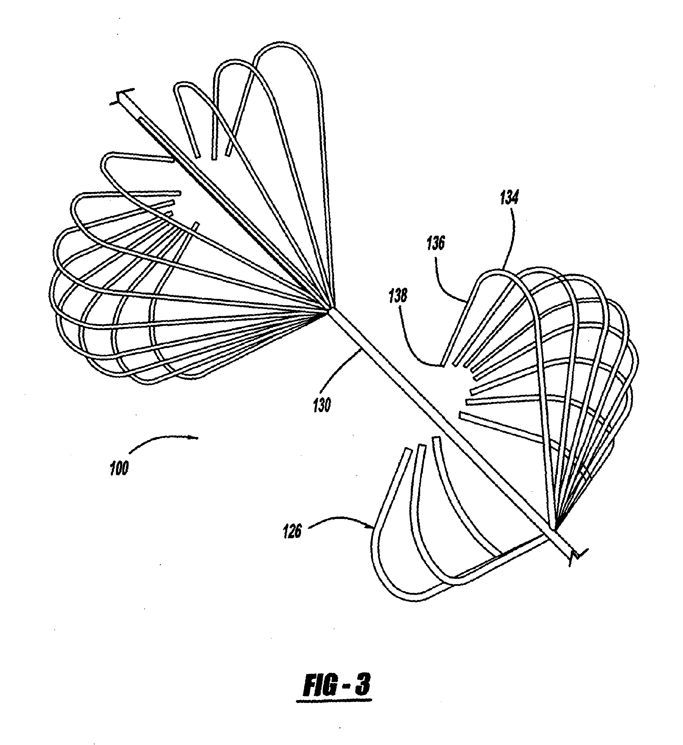

Figure 3 illustrates an alternative preferred embodiment of the present invention which is indicated generally by the numeral 100. Medical filter 100 is similar to medical filter 10 and has spine 130 but has an alternatively designed basket 126 wherein the free end segments 136 of arms 134 extend radially inwardly providing generally transversely oriented filter segments. Thus, truncated filter baskets 100 are well adapted to prevent filtered material from dislodging.

Retrieval element 32 can be formed as one piece with spine 12 or can be a separate piece attached to spine 12. Retrieval element 32 can be a retrieval hook or any other element adapted to facilitate retrieval of medical filter 10. Retrieval element 32 can be a single hook as shown in Figure 2 or can have a T-shape with twin hooks or any other suitable shape which can operatively interact with a snare device or any other suitable element which will operatively interact with a retrieval means. It will be appreciated by those skilled in the art that hooks and snares are well-known and that the particular retrieval means employed is subject to variation within the scope of the present invention.

Filter 10 may be made of any suitable material using a variety of methods. Nitinol is a preferred material but elgiloy, cobalt chromium, stainless steel or suitable plastic are examples of other materials that may be used so long as the material has the desired characteristics of strength, resilience, flexibility, biocompatibility and endurance and is suitable for the particular manufacturing technique employed. It is, of course, required that the material employed be capable of expanding to the desired shape upon ejection from the delivery catheter. Thus, the material must also be sufficiently resilient to accomplish both compression in the delivery catheter and expansion upon ejection from the catheter.

Suitable methods of manufacture of medical filter 10 include cutting a pattern into a tube to enable expansion of the tube into the desired body and struts. Another suitable method is forming the struts and body from separate strips or wires and then joining the respective parts together by suitable methods which are well known in the art.

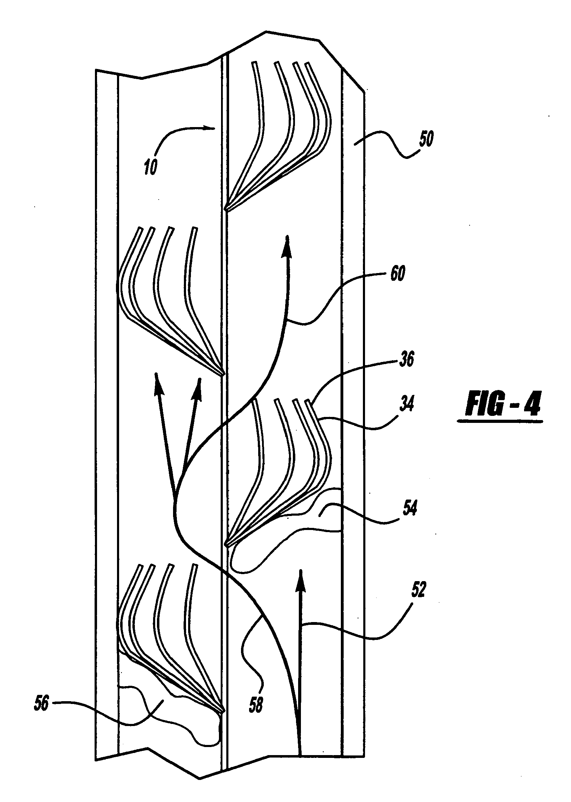

Referring to Figure 4, medical filter 10 is illustrated in use in blood vessel 50. The direction of flow of the blood stream is indicated by arrow 52. Clots which have been trapped are indicated by numerals 54 and 56. As is well illustrated in the Figure 4, the blood flow 52 is substantially unimpeded by filter 10 even though baskets 14 and 16 hold clots 54 and 56. Furthermore, any clots that bypass a filled basket are directed as shown by arrows 58 and 60 to the next available basket for entrapment. Thus, medical filter 10 has a low risk of occluding. It is contemplated that filter 10 will be preferably oriented with the "open" ends of the truncated filter baskets facing downstream. Thus, clots or other unwanted thrombus or other material to be filtered will first encounter the "closed" ends of the truncated filter baskets and tend to be forced radially outwardly toward the wall of the blood vessel where they will tend to become trapped.

The inward direction of free end segments 36 of arms 34 facilitate removal of filter 10 from an associated vessel or body passage. Thus, filter 10 has arms with no closed elements which will be in contact with a vessel wall and is thus designed for long term retrievability. Thus, although filter 10 is suitable for permanent placement in a vessel or body passage, filter 10 may be readily removed or retrieved if retrieval is desired. Retrieval of filter 10 may be accomplished by means of a conventional retrieval catheter which may be inserted from the appropriate direction to approach the end of filter 10 having retrieval element 32. After snaring retrieval element 32 with a snare, filter 10 is drawn into the lumen of the retrieval catheter and retrieved in a conventional manner. It will be appreciated that the free ends 36 of arms 34 allow removal of arms 34 from any tissue that may have grown around them with minimal trauma.

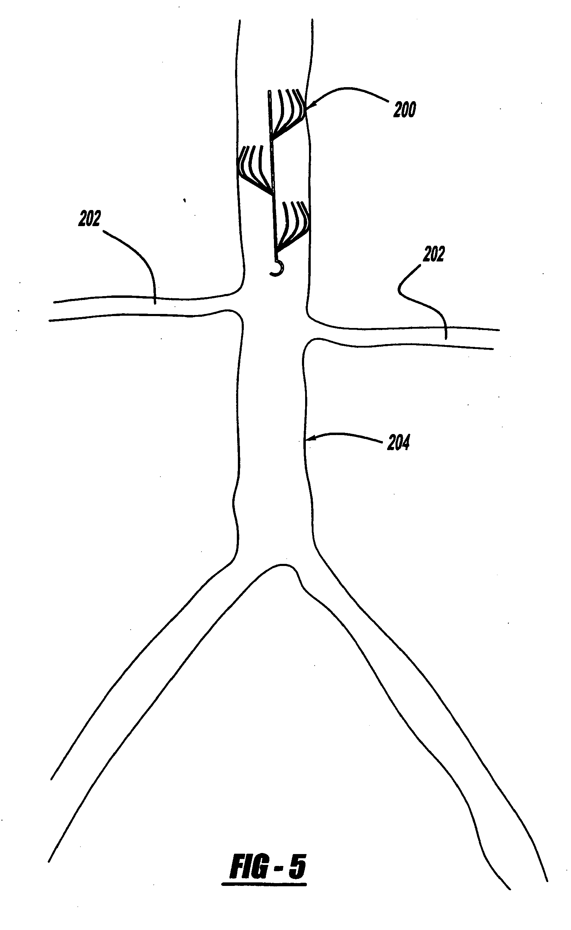

Referring to Figures 5-7, further advantages of use of the present invention are illustrated. In Figure 5, a short version having only three truncated baskets is shown and indicated by then numeral 200. Filter 200 is illustrated in place above (or below) the renal veins 202 of the Inferior Vena Cava 204. Generally speaking such use of medical filters would be not advisable because of the risk associated with occlusion but in appropriate circumstances a filter of the present invention can be so employed because of its resistance to occlusion.

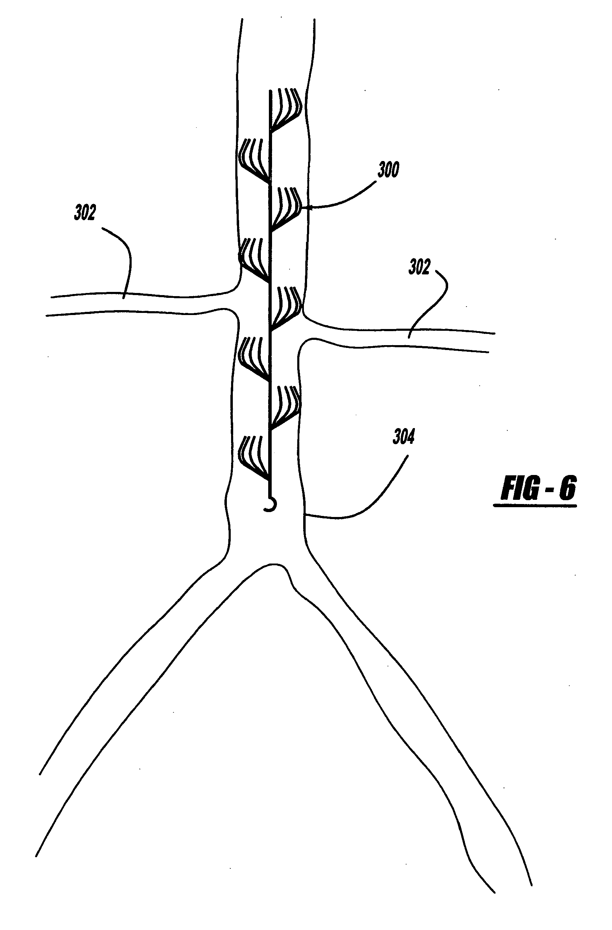

Figure 6 illustrates a long version of a preferred embodiment of the present invention, indicated generally by the numeral 300. Medical filter 300 is shown in position across renal veins 302 of the Inferior Vena Cava 304 and can be so placed because of its resistance to occlusion.

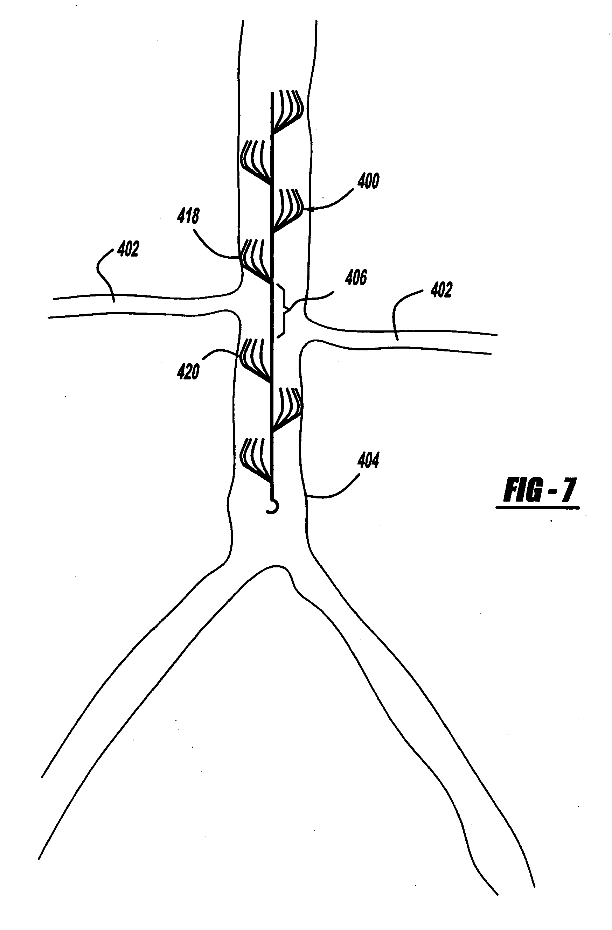

Figure 7 illustrates a further variation of a preferred embodiment of the present invention wherein medical filter 400 is shown in place above and below renal veins 402 of Inferior Vena Cava 404. Filter 400 incorporates an selected distance between baskets 418 and 420 so as not to interfere with the renal arteries.

It will be appreciated that the present invention offers several advantages over conventional medical filters. The present invention provides a filter that captures clots or other filtered material over a longer length that conventional filters and this increases the filter efficiency while decreasing the risk of caval occlusion. The filter can be placed above or across the renal veins in the inferior vena cava. The filter can be left in place until there is no visible thrombus formation on the filter. The length of the filter offers increased resistance to migration after it is in place. The length and construction of the filter also makes it suitable for use in natural bends in the vena cava or other vessels. Space between baskets can be adjusted to eliminate interference with the renal veins or other parts of the anatomy.

While preferred embodiments of the present invention have been specifically described above, it will be appreciated by those skilled in the art that the present invention is subject to variations and modifications. For example, the filter baskets may be modified by modifying the number of arms and/or their pattern. The number of baskets may vary. The gaps between baskets may be increased or decreased. The open area of each basket may vary along the length of the filter to target specific size clots. The filter may be cut from a single piece of tubing. Extra arms may be added to the spine adjacent to the retrieval element to radiate outwardly to keep the element from the vessel wall to make it easier to snare. The retrieval element may be modified by changing the angle of the hook and/or by modifying the number of tines for engaging the retrieval snare. These and other modifications are contemplated to be within the scope of the present invention which is intended to be limited only by the following claims.

## Claims

1. An elongated medical filter which is radially expandable and compressible and is intended for placement inside a tubular body passage such as a blood vessel of a patient, the medical filter comprising in expanded form:
A. a longitudinally extending spine with a retrieval element at one end thereof;
B. a plurality of truncated filter baskets spaced longitudinally on said spine, each of truncated baskets defined by a plurality of individual arms extending radially outwardly from said spine, each of said truncated baskets being rotated about said longitudinal axis with respect to adjacent truncated filter baskets.

2. The medical filter of claim 1, wherein said arms of each truncated filter basket are radially spaced about 180 degrees about said longitudinal axis.

3. The medical filter of claim 1, wherein each of said arms has a free end extending generally axially and radially inwardly.

4. The medical filter of claim 1, wherein each of said arms extends radially inwardly at an angle approximately transverse to said longitudinal axis.

5. The medical filter of claim 1, wherein said spine is comprised of a flexible material.

6. The medical filter of claim 1, wherein said retrieval element is a hook.

7. The medical filter of claim 1, wherein said filter is comprised of Nitinol.

8. The medical filter of claim 1 wherein said filter is comprised of stainless steel.

9. The medical filter of claim 1 wherein said arms are comprised of wire.

10. The medical filter of claim 1 wherein said arms are cut from tubing.

## Patentansprüche

1. Längliches medizinisches Filter, welches radial expandierbar und komprimierbar ist und bestimmt ist für eine Platzierung in einem röhrenförmigen Körperdurchgang, wie einem Blutgefäß eines Patienten, wobei das medizinische Filter in der expandierten Form Folgendes umfasst:
A. ein sich longitudinal erstreckendes Rückgrat mit einem Rückholelement an einem Ende davon;
B. mehrere gestutzte Filterkörbe, welche mit longitudinalem Abstand zueinander auf dem Rückgrat angeordnet sind, wobei jeder der gestutzten Körbe von einer Vielzahl einzelner Arme definiert ist, welche sich radial von dem Rückgrat nach außen erstrecken, wobei jeder der gestutzten Körbe bezüglich benachbarter gestutzter Filterkörbe um die longitudinale Achse gedreht ist.

2. Medizinisches Filter nach Anspruch 1, wobei die Arme jedes gestutzten Filterkorbes radial um etwa 180° um die longitudinale Achse herum angeordnet sind.

3. Medizinisches Filter nach Anspruch 1, wobei jeder der Arme ein freies Ende aufweist, welches sich im Allgemeinen axial und radial nach innen erstreckt.

4. Medizinisches Filter nach Anspruch 1, wobei sich jeder der Arme bei einem Winkel radial nach innen erstreckt, welcher annähernd quer zu der longitudinalen Achse ist.

5. Medizinisches Filter nach Anspruch 1, wobei das Rückgrat ein flexibles Material umfasst.

6. Medizinisches Filter nach Anspruch 1, wobei das Rückholelement ein Haken ist.

7. Medizinisches Filter nach Anspruch 1, wobei das Filter Nitinol umfasst.

8. Medizinisches Filter nach Anspruch 1, wobei das Filter rostfreien Stahl umfasst.

9. Medizinisches Filter nach Anspruch 1, wobei die Arme einen Draht umfassen.

10. Medizinisches Filter nach Anspruch 1, wobei die Arme aus einem Rohrmaterial geschnitten sind.

## Revendications

1. Filtre médical allongé qui peut être déployé radialement et comprimé et est destiné à être placé à l'intérieur d'un passage corporel tubulaire tel qu'un vaisseau sanguin d'un patient, le filtre médical comprenant, dans sa forme déployée :
A. une arête s'étendant longitudinalement comprenant un élément de récupération à une extrémité de celle-ci ;
B. une pluralité de paniers filtres tronqués espacés longitudinalement sur ladite arête, chacun des paniers tronqués étant défini par une pluralité de bras individuels s'étendant radialement vers l'extérieur à partir de ladite arête, chacun desdits paniers tronqués tournant autour dudit axe longitudinal par rapport aux paniers de filtre adjacents.

2. Filtre médical selon la revendication 1, dans lequel lesdits bras de chaque panier filtre tronqué sont espacés radialement d'environ 180 degrés autour dudit axe longitudinal.

3. Filtre médical selon la revendication 1, dans lequel chacun desdits bras comporte une extrémité libre s'étendant généralement axialement et radialement vers l'intérieur.

4. Filtre médical selon la revendication 1, dans lequel chacun desdits bras s'étend radialement vers l'intérieur selon un angle approximativement transversal par rapport audit axe longitudinal.

5. Filtre médical selon la revendication 1, dans lequel ladite arête est constituée d'un matériau souple.

6. Filtre médical selon la revendication 1, dans lequel ledit élément de récupération est un crochet.

7. Filtre médical selon la revendication 1, dans lequel ledit filtre est constitué de Nitinol.

8. Filtre médical selon la revendication 1, dans lequel ledit filtre est constitué d'acier inoxydable.

9. Filtre médical selon la revendication 1, dans lequel lesdits bras sont constitués de fil.

10. Filtre médical selon la revendication 1, dans lequel lesdits bras sont découpés dans des tubes.
